# EUROPEAN PATENT APPLICATION

(11) **EP 4 019 120 A1**
(43) Date of publication of application: **29.06.2022**
(21) Application number: 20217394.4
(22) Date of filing: 28.12.2020
(51) Int. Cl.: B01D 69/02, A61L 9/04, A61L 9/12

(54) **CONTAINER FOR RELEASING VOLATILE SUBSTANCES**

(71) Applicant: Zobele Holding SpA, 38100 Trento (IT)
(72) Inventor: MORHAIN, Cedric, 38100 Trento (IT); DEFLORIAN, Stefano, 38100 Trento (IT)
(74) Representative: Herrero & Asociados, S.L.

(57) **Abstract**

Container for releasing volatile substances, comprising a container body (1) that contains a liquid with volatile substances and that is provided with an aperture (2); a porous membrane (3) that closes the aperture (2) of the container body (1); and wherein the porous membrane (3) is transparent when it is in contact with the liquid in the container body (1).

The invention provides a container for releasing volatile substances that permits to show the user that the releasing of the volatile substances is exhausted in a very simply way.

## Description

The present invention refers to a container for releasing volatile substances comprising a porous membrane that gets transparent when is in contact with a liquid inside the container.

### Background of the invention

Membrane based devices for releasing volatile substances in the atmosphere and based on membranes are known for years.

These devices comprise a container, often made by thermoforming, that is closed by the membrane, in order to let the vapor pass through it but not the liquid. On top of the membrane a barrier layer is applied. This barrier layer avoids the substance to go out during the storage and has to be removed by user before a first use.

It is a general need for commercial products to have a clear end of life, in order to communicate the user that the product is exhausted and that a new device need to be used.

The membranes are generally transparent and thus liquid can be seen. But a stronger end of life indication is anyway welcome. In other cases, a porous element is present in the container behind the membrane to act as a wick, feeding with liquid the surface of the membrane and then having maximum evaporation. In such cases, the porous element will hide the liquid and then end of life indication is lost.

### Description of the invention

Therefore, one purpose of the present invention is to provide a container for releasing volatile substances that permits to show the user that the releasing of the volatile substances is exhausted in a very simply way.

With the container for releasing volatile substances according to the present invention it is possible to solve said drawbacks, providing other advantages that are described below.

The container for releasing volatile substances according to the present invention comprises:
- a container body that contains a liquid with volatile substances and that is provided with an aperture;
- a porous membrane that closes the aperture of the container body; and
wherein the porous membrane is transparent when it is in contact with the liquid in the container body.

The transparency of the porous membrane is obtained when the liquid is inside the pores of the membrane. The porous membrane is not transparent, such as opaque, when the porous membrane is not in contact with the liquid in the container body.

The container according to the present invention can also comprise a barrier layer detachably connected to the porous membrane.

The container for releasing volatile substances according the present invention comprises a message or pattern that is viewed when the porous membrane is transparent.

The message or pattern can be printed on a side of the porous membrane, and/or can be printed on a printed support.

The printed support can be placed between the container body and the porous membrane, or between the porous membrane and the barrier layer.

Advantageously, the container body is transparent.

Preferably, the container body comprises a liquid residue area such as a cavity or a porous element.

### Brief description of the drawings

For a better understanding the above explanation and for the sole purpose of providing an example, some non-limiting drawings are included that schematically depict a practical embodiment.
Figure 1 is an elevation view in section of the container according to the present invention, according to a first embodiment, wherein the pattern is printed on the substrate;
Figure 2 is an elevation view in section of the container according to the present invention, according to a second embodiment, wherein a printed support is placed between the barrier layer and the porous membrane;
Figure 3 is an elevation view in section of the container according to the present invention, according to a third embodiment, wherein a printed support is placed between the container body and the porous membrane;
Figure 4 is a frontal perspective view of the container according to the present invention, where the complete pattern can be viewed, because the container is full of liquid;
Figure 5 is a rear perspective view of the container according to the present invention, wherein the pattern can be completely viewed, because the container is full of liquid;
Figure 6 is a rear perspective view of the container according to the present invention, wherein the pattern can be partially viewed, because the container is partially empty of liquid;
Figure 7 is an elevation view in section of the container according to the present invention, according to the first embodiment, showing a cavity as a liquid residue area; and
Figure 8 is an elevation view in section of the container according to the present invention, according to the first embodiment, showing a porous element as a liquid residue area.

### Description of a preferred embodiment

The container for releasing volatile substances according to the present invention comprises:
- a container body 1, preferably transparent, provided with an aperture 2 that contains a liquid with the volatile substances;
- a porous membrane 3 that gets transparent when is in contact with the liquid containing the volatile substances placed inside the container body; and
- optionally, a barrier layer 4 detachably connected to the porous membrane 3, and that is removed before a first use.

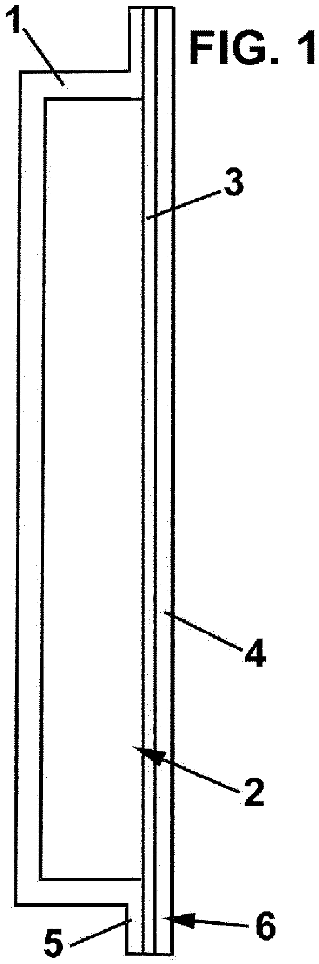

As shown in Fig. 1, the container body 1 comprises a perimetral rim 5 defining a sealing zone 6 where the porous membrane 3 is joined to the container body 1.

A message or a pattern 7 is placed behind the porous membrane 3 and is thus visible only when the membrane is transparent.

In the present application "behind" means on the opposite side of the porous membrane 3 with respect where user can look at it:
- If user is seeing the product from the front side, i.e. through the container body 1, the message will be placed on the external side of the porous membrane 3, and
- If user can only see the back of the porous membrane 3, then message is be placed on the inner side of the porous membrane 3.

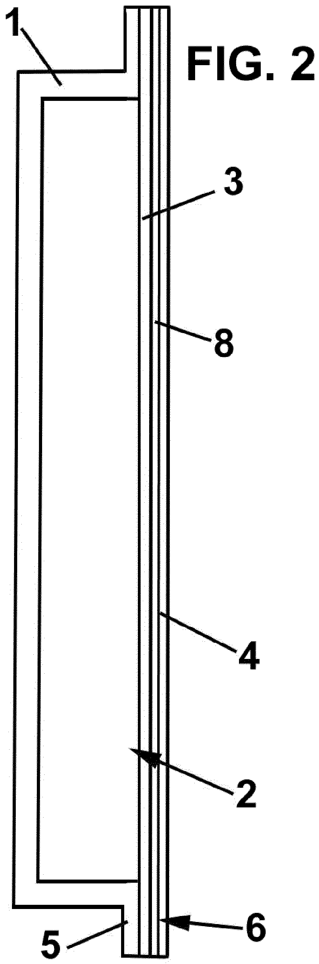

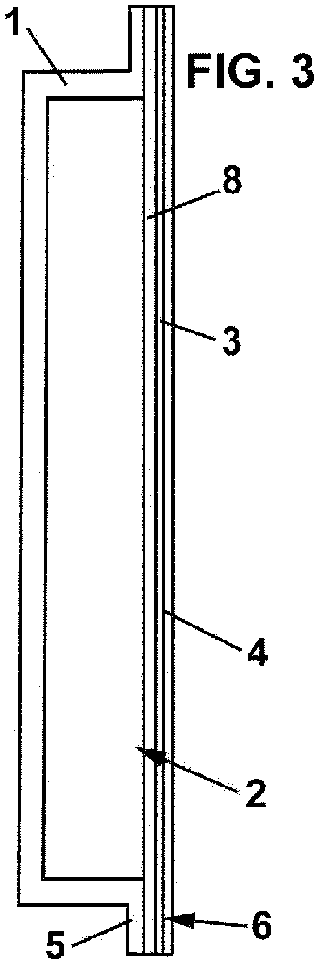

The message or pattern 7 can be made by printing the message on the behind face of the porous membrane 3 (embodiment of Fig. 1) or by placing a printed support 8 (embodiments of Figs. 2 and 3).

In addition, there a is a residue trap, often, even if optimized, fragrances have a nonvolatile part (or low volatile, i.e. a part that will evaporate very slowly without having any intensity per se). If nothing is done, this non or low volatile part avoids the porous membrane 3 to turn back to opaque during this zero or low evaporation phase.

To avoid this, a liquid residue containing area can be placed inside the container body 1. This liquid residue area needs to be separated from the porous membrane 3 (not in fluid contact when liquid is almost exhausted).

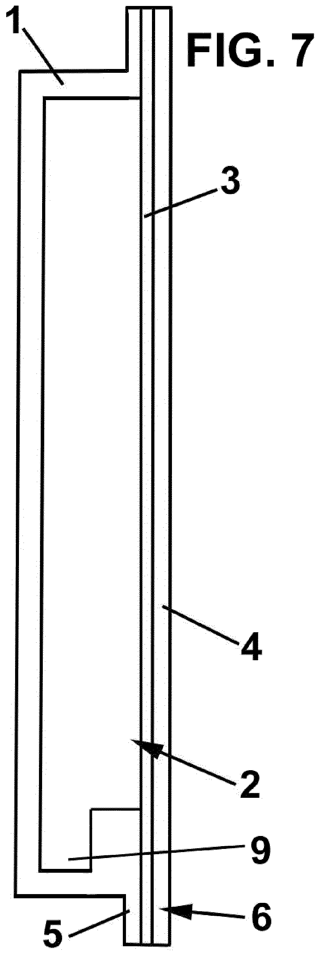

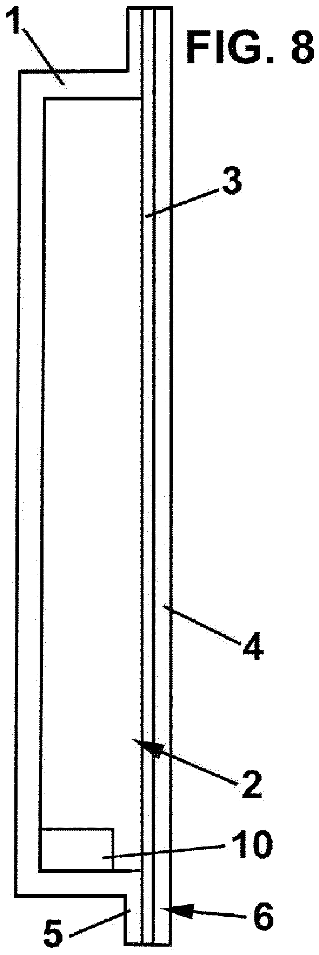

This liquid residue area can be a cavity 9 (Fig. 7) or a porous element 10 (Fig. 8). It must be pointed out that the cavity 9 and or the porous 10 can be in any of the embodiments shown in Figs. 1-3.

During the evaporation of the liquid, after the removal of the barrier layer 4, the porous membrane 3 and liquid residue area are in fluid contact as the container body 1 is full of liquid.

As more volatile substances are evaporating first, composition of the liquid is changing with higher concentration of low volatility components. When a certain amount of substance has evaporated, typically more than 90% and ideally more than 95%, the residue will be contained is the liquid residue area and will not penetrate the porous membrane 3.

The volatile substances that are in the porous membrane 3 will thus evaporate completely and porous membrane 3 will turn back to opaque.

In addition, ideally, the porous membrane 3 has a certain capillarity property: in order to have the full porous membrane 1 transparent until end of life, the liquid should be spread in the whole porous membrane 1. For this, it is necessary the capillary rise of the liquid to be at least equivalent to length of the porous membrane 3 in vertical position.

When the container is used for first time, the user must remove the barrier layer 4, permitting the releasing of the volatile substances through the porous membrane 3.

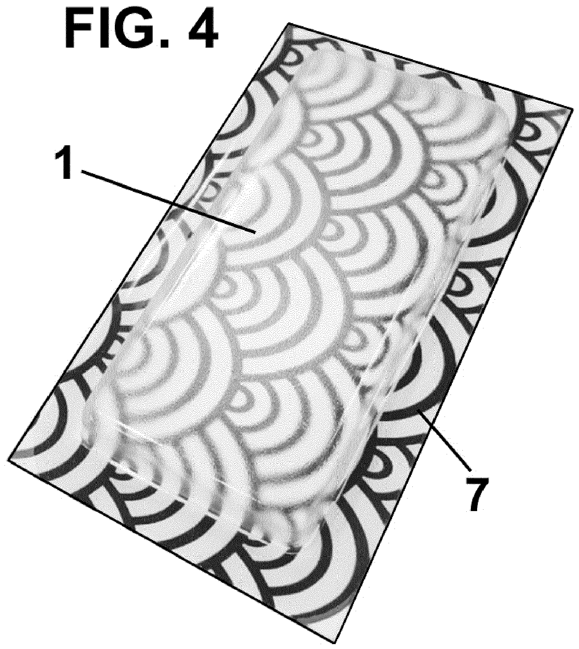

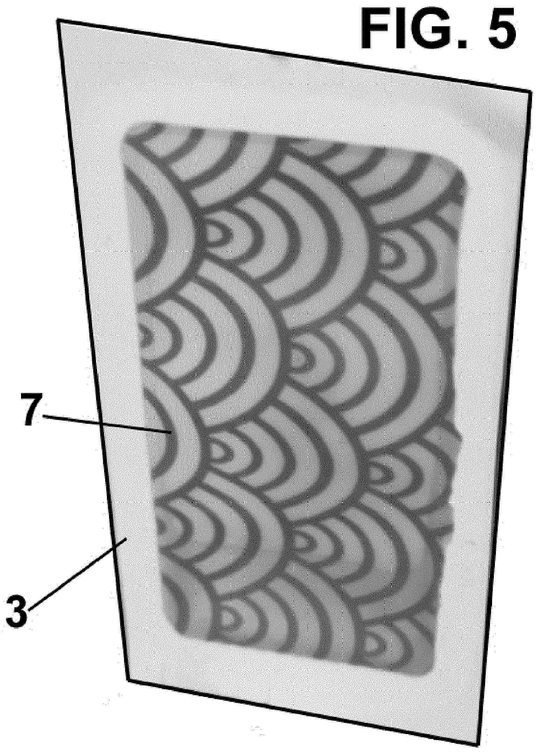

As the porous membrane 1 is in contact with the liquid, it is transparent and the user can see the message or pattern 7 (Figs. 4 and 5), indicating that the container continues the releasing of volatile substances.

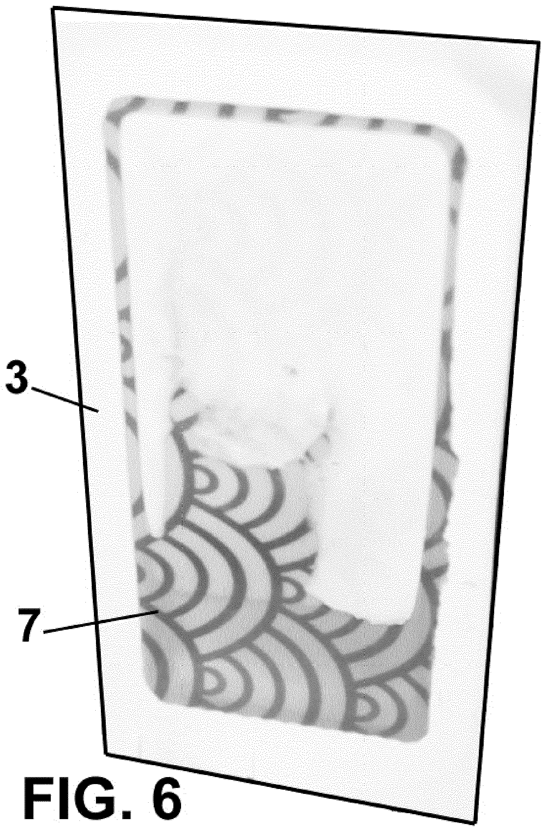

As the liquid inside the container is wasted, the porous membrane 1 loses progressively its transparency (Fig. 6), and when it is completely opaque, the user cannot see the message or pattern 7, and therefore the user knows that the liquid is exhausted.

Even though reference has been made to a specific embodiment of the invention, it is obvious for a person skilled in the art that the container for releasing volatile substances described herein is susceptible to numerous variations and modifications, and that all of the details mentioned can be substituted for other technically equivalent ones without departing from the scope of protection defined by the attached claims.

## Claims

1. Container for releasing volatile substances, comprising:
- a container body (1) that contains a liquid with volatile substances and that is provided with an aperture (2);
- a porous membrane (3) that closes the aperture (2) of the container body (1); and **characterized in that** the porous membrane (3) is transparent when it is in contact with the liquid in the container body (1).

2. Container for releasing volatile substances according to claim 1, that also comprises a barrier layer (4) detachably connected to the porous membrane (3).

3. Container for releasing volatile substances according to claim 1, that comprises a message or pattern (7) that is viewed when the porous membrane (3) is transparent.

4. Container for releasing volatile substances according to claim 3, wherein the message or pattern (7) is printed on a side of the porous membrane (3).

5. Container for releasing volatile substances according to claim 3, wherein the message or pattern (7) is printed on a printed support (8).

6. Container for releasing volatile substances according to claim 5, wherein the printed support (8) is placed between the container body (1) and the porous membrane (3).

7. Container for releasing volatile substances according to claim 5, wherein the printed support (8) is placed between the porous membrane (3) and the barrier layer (4).

8. Container for releasing volatile substances according to claim 1, wherein the container body (1) is transparent.

9. Container for releasing volatile substances according to anyone of the previous claims, wherein the container body (1) comprises a liquid residue area (9, 10).

10. Container for releasing volatile substances according to claim 9, wherein the liquid residue area is a cavity (9).

11. Container for releasing volatile substances according to claim 9, wherein the liquid residue area is a porous element (10).
